# EUROPEAN PATENT APPLICATION

(11) **EP 2 551 273 A1**
(43) Date of publication of application: **30.01.2013**
(21) Application number: 11734658.5
(22) Date of filing: 19.01.2011
(51) Int. Cl.: C07F 9/6561, C07H 19/167, C07H 21/02

(54) **PHOSPHORYLATION REAGENT**

(30) Priority: 20.01.2010 JP 2010010260
(71) Applicant: The University of Tokyo, Bunkyo-Ku Tokyo 113-8654 (JP); Nippon Shinyaku Co., Ltd., Kyoto-shi Kyoto 601-8550 (JP)
(72) Inventor: WADA, Takeshi, Tokyo 113-8654 (JP); NAGATA, Seigo, Tsukuba-shi Ibaraki 305-0032 (JP); UETAKE, Koichi, Otsu-shi Shiga 520-2144 (JP)
(74) Representative: Vossius & Partner
(86) International application number: PCT/JP2011/050822
(87) International publication number: WO 2011/090052

(57) **Abstract**

Disclosed is a novel phosphorylating reagent.

Specifically disclosed is a compound represented by general formula (A). 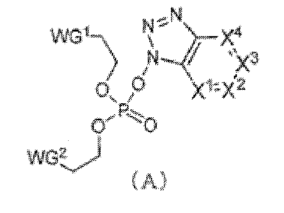 In the formula, WG¹ and WG² independently represent a cyano group, a nitro group, a halogen atom, an alkylsulfonyl group or an arylsulfonyl group; X¹, X², X³ and X⁴ independently represent CR or N; and R represents H, a methyl group, a halogen atom, a trifluoromethyl, group, a cyano group, a nitro gtoup, an alkoxycarbonyl group, a carbamoyl group, a monoalkylcarbamoyl group, a dialkylcarbamoyl group, a sulfamoyl group, a monoalkylsulfamoyl group, a dialkylsulfamoyl group, or an alkylsulfonyl group.

## Description

### [Technical Field]

The present invention relates to a novel phosphorylating reagent.

### [Background Art]

To synthesize a pyrophosphorylated derivative of a nucleic acid, it is necessary to synthesize a nucleic acid of the desired chain length on a solid support, to phosphorylate the nucleic acid at the 5'-terminal hydroxyl group, and then to further phosphorylate the monophosphorylated derivative. Generally in the phosphorylation of a hydroxyl group of a nucleic acid (monophosphorylation), a phosphoramidite compound is employed. However, the phosphorylation of a monophosphorylated derivative using a phosphoramidite compound is a low-yield reaction.
While a compound represented by the following chemical formula having a phosphoric acid diester structure has already been reported as a reagent for phosphorylating the monophosphorylated derivative of a nucleic acid (see Non-patent literature 1), no compound having a phosphoric acid triester structure has been reported.

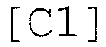

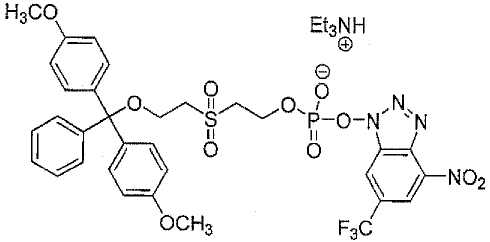

### [Citation List]

### [Non-patent Literature]

[Non-patent Literature 1] Sekine et al., Tetrahedron Lett., 2001, Vol.42, p.8853-8856

### [Summary of Invention]

### [Problem to be Solved by Invention]

An object of the present invention is mainly to provide a novel phosphorylating reagent.

### [Means for Solving Problem]

The present inventors have discovered that a compound represented by the following formula (A) is useful as a phosphorylating reagent and have established the present invention. The present invention may for example be the following 1 to 5.
1. A compound represented by the following formula (A): 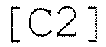 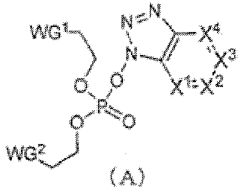 wherein WG¹ and WG² are the same or different and each represents cyano, nitro, halogen, alkylsulfonyl optionally substituted with hydroxy protected by a group represented by the following formula (1) or arylsulfonyl optionally substituted with 1 to 3 groups selected from the group of halogen, alkyl, alkoxy, cyano and nitro; X¹, X², X³ and X⁴ are the same or different and each represents CR or N; and R represents H, methyl, halogen, trifluoromethyl, cyano, nitro, alkoxycarbonyl, carbamoyl, monoalkylcarbamoyl, dialkylcarbamoyl, sulfamoyl, monoalkylsulfamoyl, dialkylsulfamoyl or alkylsulfonyl; 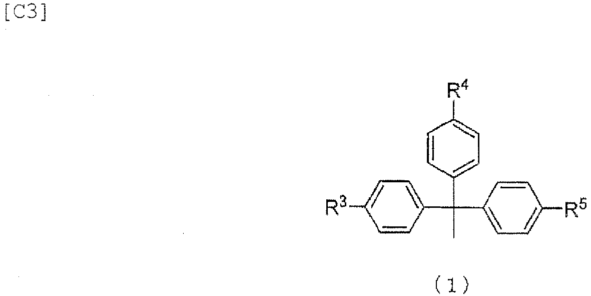 wherein R³, R⁴ and R⁵ are the same or different and each represents hydrogen or alkoxy.
2. A phosphorylating reagent containing a compound described in 1 shown above.
3. A method for producing a compound represented by the following formula (A), including reacting a compound represented by the following formula (2) with a compound represented by the following formula (3): 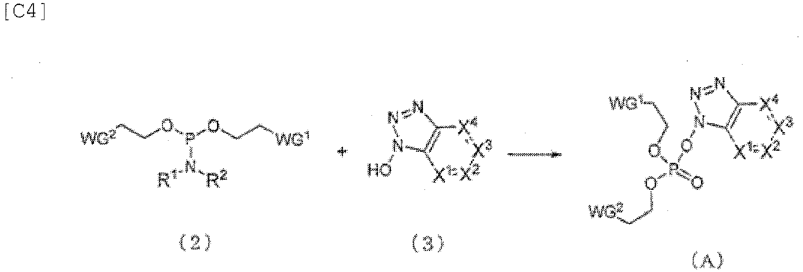 wherein WG¹, WG², X¹, X², X³ and X⁴ are defined as described above, and R¹ and R² are the same or different and each represents alkyl.
4. A method for producing a compound represented by the following formula (5), including reacting a compound represented by the following formula (4) with a compound represented by the following formula (A): 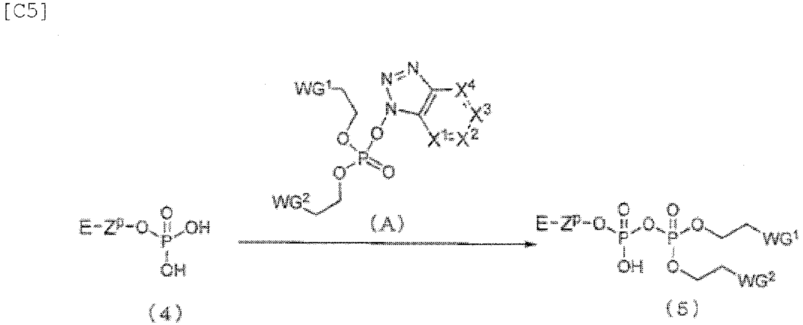 wherein E represents a solid support; Z^{p} represents an optionally protected nucleic acid; and WG¹, WG², X¹, X², X³ and X⁴ are defined as described above.
5. A method for producing a compound represented by the following formula (9), including the following Step 1 to Step 3: 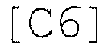 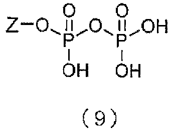 wherein Z represents a nucleic acid;
   (Step 1) a step for reacting a compound represented by the following formula (4) with a compound represented by the following formula (A) to produce a compound represented by the following formula (5): 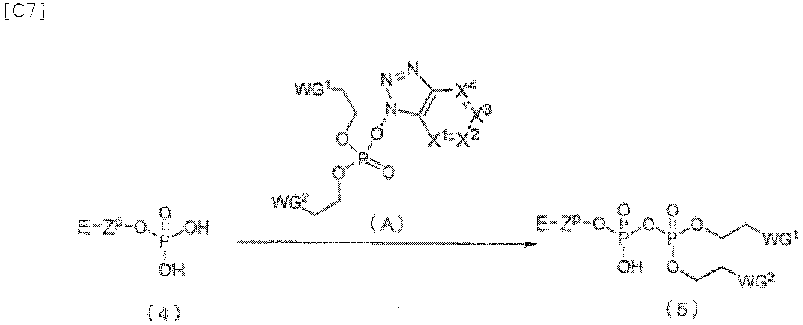 wherein E, WG¹, WG², X¹, X², X³, X⁴ and Z^{p} are defined as described above; (Step 2) a step for reacting Compound (5) with a silylating agent represented by the following formula (6) and a base to produce a compound represented by the following formula (7): 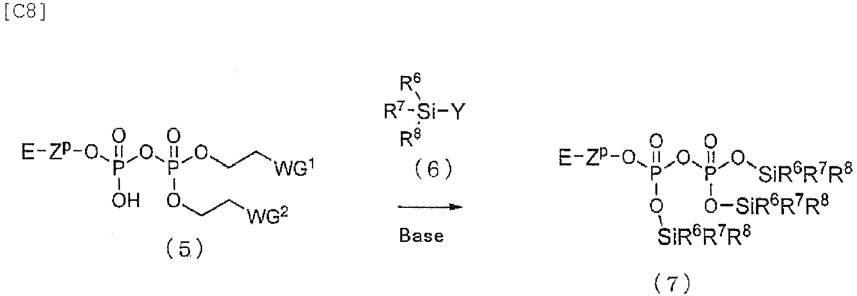 wherein E, WG¹, WG² and Z^{p} are defined as described above; R⁶, R⁷ and R⁸ are the same or different and each represents alkyl, phenyl or naphthyl; and Y represents halogen, trifluoromethanesulfonyloxy or a group represented by the following formula (8): 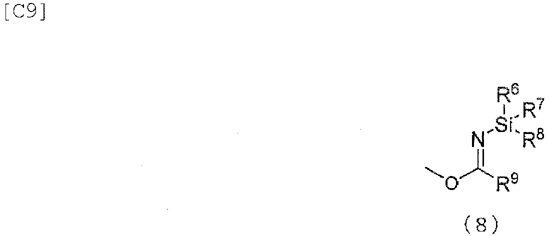 wherein R⁶, R⁷ and R⁸ are defined as described above; and R⁹ represents alkyl; (Step 3) a step for removing all protecting groups from Compound (7) to produce Compound (9); 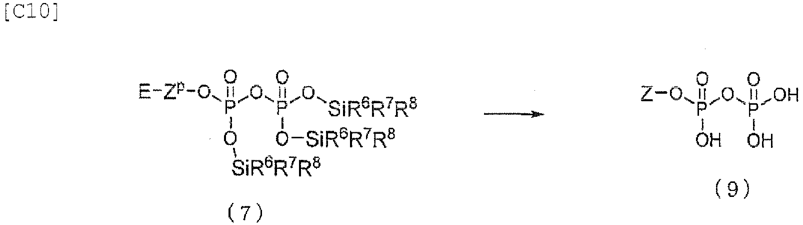
wherein E, R⁶, R⁷, R⁸ and Z^{p} are defined as described above; and Z represents a nucleic acid.

The following is a detailed description of the terms employed in this specification.
As a "solid support", any of those which can be used in a solid-phase synthesis such as for nucleic acids, peptides, peptide nucleic acids, saccharides and the like can generally be employed with no specific consideration, and it may for example be a controlled pore glass (CPG), an oxalyl-controlled pore glass (see for example Alul et al., Nucleic Acids Research, Vol. 19, 1527 (1991)), TentaGel support-aminopolyethylene glycol derivatized support (for example, see Wright et al., Tetrahedron Letters, Vol. 34, 3373 (1993)), a Poros-polystyrene/divinylbenzene copolymer, a polystyrenic resin and a polyacrylamide-based resin.
The "nucleic acid" relating to Z and Z^{p} may for example be a polynucleotide formed from 2 to 200 nucleosides as a result of binding via a phosphorus bond (for example, phosphate bond, phosphorothioate bond, phosphorodithioate bond, alkylphosphonate bond, boranophosphate bond or phosphoramidate bond) between the 3'-position and the 5'-position of adjacent nucleosides.
The "nucleoside" may for example be deoxyribonucleoside, ribonucleoside, a modified nucleoside represented by the following formula (10) or (11); 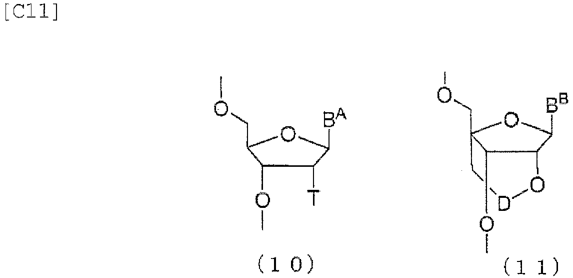 wherein each of B^{A} and B^{B} represents independently an optionally substituted nucleic acid base; T represents alkyl, alkoxy, alkoxyalkyl, halogen, nitro and cyano; and D represents a single bond, methylene, ethylene, -N(CH₃)- or -OCH₂- (for example, see Singh et al., Chem. Commun., 1998, 4:455-456; Koshkin et al., Tetrahedron, 1998, 54:3607-3630, WO03/068795, WO2005/021570).

The "nucleic acid base" is not particularly limited as long as it is employed in synthesizing a nucleic acid, and it may for example be a pyrimidine base such as cytosine, uracil, thymine and the like, or a purine base such as adenine, guanine, hypoxanthine and the like. The "nucleic acid base" may be substituted in any substitutable position. Such a substituent may for example be a halogen, acyl, alkyl, arylalkyl, alkoxy, hydroxy, amino, monoalkylamino; dialkylamino, carboxy, cyano and nitro, and 1 to 3 of these groups may serve as substituents.

The nucleic acid relating to Z^{p} may allow a nucleoside (nucleic acid base, saccharide moiety) or a phosphorus bond moiety (for example, phosphate bond, phosphorothioate bond, phosphorodithioate bond, alkylphosphonate bond, boranophosphate bond or phosphoramidate bond) to be protected, and a "nucleic acid base" may allow the amino group of adenine, guanine or cytosine to be protected. Here an amino protecting group is not particularly limited as long as it is employed as a protecting group for a nucleic acid base, and it may for example be benzoyl, 4-methoxybenzoyl, acetyl, propionyl, butyryl, isobutyryl, phenylacetyl, phenoxyacetyl, 4-tert-butylphenoxyacetyl, 4-isopropylphenoxyacetyl and (dimethylamino)methylene. Those preferred especially as protecting groups for the amino group of guanine are phenoxyacetyl, 4-t-butylphenoxyacetyl and 4-isopropylphenoxyacetyl. In the "saccharide moiety", the 2'-hydroxyl group of the ribose may be protected, and such a protecting group for the 2'-hydroxyl group of the ribose may for example be acyl, trisubstituted silyl, trisubstituted silyloxymethyl, 2-cyanoethoxymethyl, 2-nitroethoxymethyl, 2-halogenoethoxyethyl, 2-(arylsulfonyl)ethoxyethyl, 2-(alkylsulfonyl)ethoxyethyl, or 2-cyanoethoxy-2-ethyl. The "phosphorus bond moiety" may also be protected and the protecting group for such a phosphorus bond moiety may for example be cyanoethyl.

The "alkyl" may for example be a straight-chain or branched-chain alkyl having 1 to 5 carbon atoms. Those exemplified typically are methyl, ethyl, n-propyl, isopropyl, n-butyl, isobutyl, sec-butyl, tert-butyl, n-pentyl, isopentyl, neopentyl and tert-pentyl.

The "alkyl" moiety of "monoalkylcarbamoyl", "dialkylcarbamoyl", "monoalkylsulfamoyl", "dialkylsulfamoyl", "alkylsulfonyl", "2-(alkylsulfonyl)ethoxyethyl", "arylalkyl", "monoalkylamino" and "dialkylamino" may for example be those similar to the aforementioned "alkyl".

The "alkoxy" may for example be a straight-chain or branched-chain alkoxy having 1 to 4 carbon atoms. Those exemplified typically are methoxy, ethoxy, n-propoxy, isopropoxy, n-butoxy, isobutoxy, sec-butoxy, tert-butoxy and the like. Among these, ones having 1 to 3 carbon atoms are preferred and methoxy is especially preferred.

The "alkoxy" moiety of the "alkoxyalkyl" and "alkoxycarbonyl" may for example be those similar to the aforementioned "alkoxy".

The "halogen" may for example be fluorine, chlorine, bromine and iodine.

The "halogen" moiety of the "2-halogenoethoxyethyl" may for example be those similar to the aforementioned "halogen".

The "aryl" moiety of the "arylalkyl", "arylsulfonyl" and "2-(arylsulfonyl)ethoxyethyl" may for example be an aryl having 6 to 12 carbon atoms. Those exemplified typically are phenyl, 1-naphthyl, 2-naphthyl and biphenyl. The said aryl may optionally be substituted, and such a substituent may for example be a halogen, alkyl, alkoxy, cyano and nitro, and 1 to 3 of these groups may serve as substituents in any positions.

The "acyl" may for example be a straight-chain or branched-chain alkanoyl having 1 to 6 carbon atoms and an aroyl having 7 to 13 carbon atoms. Those exemplified typically are formyl, acetyl, n-propionyl, isopropionyl, n-butyryl, isobutyryl, tert-butyryl, valeryl, hexanoyl, benzoyl, naphthoyl, levulinyl and the like.

The "trisubstituted silyl" may for example be trimethylsilyl, triethylsilyl, tri-n-propylsilyl, triisopropylsilyl, tri-n-butylsilyl, triisobutylsilyl, tri-t-butylsilyl, triphenylsilyl, tribenzylsilyl, tri-p-xylylsilyl, t-butyldimethylsilyl, t-butyldiethylsilyl, t-butyldiphenylsilyl, dimethylisopropylsilyl, diethylisopropylsilyl, dimethylthexylsilyl and diphenylmethylsilyl.

The "trisubstituted silyl" moiety in relation to the "trisubstituted silyloxymethyl" may for example be those similar to the aforementioned "trisubstituted silyl".

### [Mode for carrying out the Invention]

The embodiments of the present invention are described as follows.
In the production methods described below, when a starting material has a substituent having any effect on a reaction (for example, hydroxy, amino, carboy), then it is appropriate for the reaction to be performed after the starting material has been protected by a suitable protecting group according to a known method. Such a protecting group can finally be removed by a known method such as a catalytic reduction, an alkali treatment, an acid treatment and the like.

### I. Compound (A)

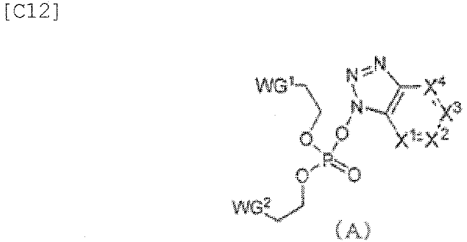

wherein WG¹, WG², X¹, X², X³ and X⁴ are defined as described above.

A preferred embodiment of Compound (A) may for example be a compound in which WG¹ and WG² are the same or different and each represents cyano or 2-(4,4'-dimethoxytrityloxy)ethylsulfonyl.

Another preferred embodiment of Compound (A) may for example be a compound in which each of X¹, X², X³ and X⁴ is CH, a compound in which each of X¹, X³ and X⁴ is CH and X² is CR, and R is trifluoromethyl, and a compound in which X¹ is N and each of X², X³ and X⁴ is CH.

Compound (A) is useful as a reagent (phosphorylating reagent) for introducing a phosphate group (-OP(=O) (OH)₂) into, for example, a compound having a hydroxyl group or an amino group.
Furthermore, the phosphorylating reagent containing Compound (A) is suitable for the efficient synthesis not only of a nucleic acid pyrophosphorylated at the 5'-end from a nucleic acid monophosphorylated at the 5'-end, but also of, for example, peptide nucleic acids, morpholino nucleic acids, peptides (see the literature relating to the production of monophosphorylated derivatives of peptides, for example, ORGANIC LETTERS, VOL. 4, No. 17, pp. 2865-2868) pyrophosphorylated at the 5'-end from the respective derivatives monophosphorylated at the 5'-end.

### II. Synthesis of Compound A

Compound (A) can be produced by the following reaction. 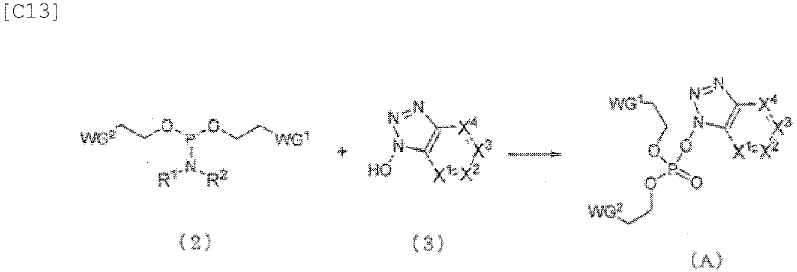 wherein WG¹, WG², R¹, R², X¹, X², X³ and X⁴ are defined as described above.

This reaction can be performed by treating Compound (3) with Compound (2).

A suitable amount of Compound (3) to be employed is in the range of 2 to 20 times the molar amount of Compound (2), and preferably in the range of 4 to 10 times the molar amount of Compound (2). The solvent which can be employed in this reaction may for example be acetonitrile, tetrahydrofuran (THF), a mixed solvent of acetonitrile and 1-methyl-2-pyrrolidone and a mixed solvent of THF and 1-methyl-2-pyrrolidone. A suitable reaction temperature may for example be within the range of 0 to 60°C. While the reaction time may vary depending on the types of starting materials employed, the reaction temperature and the like, a time period within the range of 5 minutes to 2 hours is usually suitable.

### III. Production of pyrophosphorylated derivative

Compound (9) can be produced by the following Step 1 to Step 3; 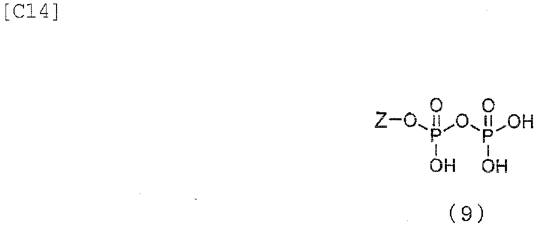 wherein Z is defined as described above.

### Step 1

A step for reacting Compound (4) with Compound (A) to produce Compound (5). 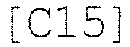 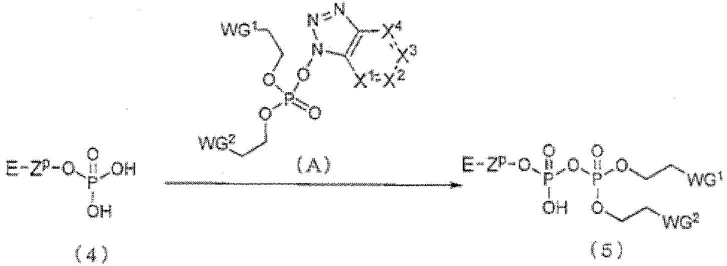 wherein E, WG¹ WG², X¹, X², X³, X⁴ and Z^{p} are defined as described above.

This step can be performed by reacting monophosphorylated Compound (4) with Compound (A).
A suitable amount of Compound (A) to be employed is in the range of 10 to 1000 times the molar amount of Compound (4), and preferably in the range of 40 to 160 times the molar amount of Compound (4). The solvent which can be employed in this reaction may for example be acetonitrile, THF, a mixed solvent of acetonitrile and 1-methyl-2-pyrrolidone and a mixed solvent of THF and 1-methyl-2-pyrrolidone. A suitable reaction temperature may for example be within the range of 0 to 60°C. While the reaction time may vary depending on the types of starting materials employed, the reaction temperature and the like, a time period within the range of 1 minute to 1 hour is usually suitable.
Compound (4) as a starting compound can be produced by monophosphorylating a nucleic acid which is synthesized according to a known method (Nucleotides, 20(3):197-212(2001)) and whose 5'-terminus is deprotected (see for example Nucleic Acids Res., 35(10):3287-3296(2007), Helv. Chim. Acta., 84:3775-3795(2001), J. Am. Chem. Soc., 120:11820-11821(1998), Proc. Natl. Acad. Sci. USA, 85:5764(1988), Singh et al., Chem. Commun. , 1998, 4:455-456; Koshkin et al., Tetrahedron, 1998, 54:3607-3630, WO03/068795, WO2005/021570).

### Step 2

A step for reacting Compound (5) with a silylating agent represented by the following formula (6) and a base to produce Compound (7). 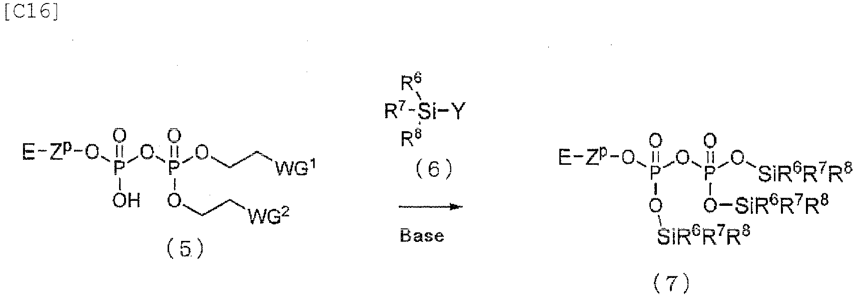 wherein E, R⁶, R⁷, R⁸, WG¹, WG², Y and Z^{p} are defined as described above.

This step can be performed by reacting Compound (5) with a silylating agent and a base.

A suitable amount of the silylating agent (6) to be employed is in the range of 100 to 10000 times the molar amount of Compound (5), and preferably in the range of 500 to 2000 times the molar amount of Compound (5).

While the base which can be employed in this step may be any of those known to those skilled in the art, one selected from the group of 1,8-diazabicyclo[5.4.0]undec-7-ene (DBU), 1,4-diazabicyclo[2.2.2]octane (DABCO) and a combination of benzylthiol and triethylamine is preferred.

A suitable amount of the base to be employed is in the range of 100 to 10000 times the molar amount of Compound (5), and preferably in the range of 1000 to 3000 times the molar amount of Compound (5).

The solvent which can be employed in this step may for example be pyridine. A suitable reaction temperature may for example be within the range of 0 to 60°C. While the reaction time may vary depending on the types of starting materials employed, the reaction temperature and the like, a time period within the range of 1 minute to 1 hour is usually suitable.

### Step 3

A step for producing Compound (9) from Compound (7); 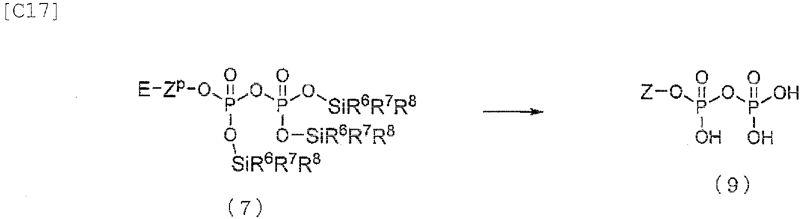 wherein E, R⁶, R⁷, R⁸, Z^{p} and Z are defined as described above.

This step is a step for cleaving Compound (7) from a solid support and removing the protecting groups according to a known method (in the case where Z is a nucleic acid; see for example Nucleic Acids Res., 35 (10):3287-3296 (2007), Helv. Chim. Acta., 84:3775-3795 (2001), J. Am. Chem. Soc., 120: 11820-11821(1998), Proc. Natl. Acad. Sci. USA, 85:5764 (1988), Singh et al., Chem. Commun., 1998, 4: 455-456; Koshkin et al., Tetrahedron, 1998, 54: 3607-3630, WO03/068795, WO2005/021570).

### [Brief Description of Drawings]

[Fig. 1] Figure 1 shows a chromatogram obtained by HPLC analysis. In this figure, the ordinate represents the absorption intensity, while the abscissa represents the retention time (minutes).
[Fig. 2] Figure 2 shows a spectrum obtained by MALDI-TOF-MS analysis. In this figure, the ordinate represents the ion intensity, while the abscissa represents the molecular weight (m/z).
[Fig. 3] Figure 3 shows a chromatogram obtained by HPLC analysis. In this figure, the ordinate represents the absorption intensity, while the abscissa represents the retention time (minutes).

### [Examples]

The present invention is described in further detail in the following Reference Examples, Examples and Comparatives, to which the invention is not limited. The HPLC conditions employed in Reference Examples, Examples and Comparatives are as shown below.

### HPLC conditions:

### HPLC Instrument

Pumping unit: LC-20AD (Manufactured by Shimadzu Industries)
Detector: SPD-20A (Manufactured by Shimadzu Industries)
Autosampler: SIL-20AC (Manufactured by Shimadzu Industries)
Column oven: CTO-20AC (Manufactured by Shimadzu Industries) Ion-exchange HPLC column
DNAPac (trade mark) PA-100 (4 mm × 250 mm) (manufactured by Dionex Corporation)
Column temperature: 50°C

### Mobile phase

Gradient: Linear gradient for 20 minutes (Solution B: 0% to 10%)
Solution A: 25 mM Tris HCl buffer solution containing 10% acetonitrile
   Solution B: 25 mM Tris HCl buffer solution containing 10% acetonitrile and
700 mM sodium perchlorate
Flow rate of mobile phase: 1.5 ml/minute
Detection wavelength: 260 nm

### Reference Example 1

### Production of 3-mer (AUA) monophosphorylated at the 5'-end (SEQ ID NO: 1)

A commercially available CPG solid support derivatized with N⁶-benzoyl-5'-O-(4,4'-dimethoxytrityl)-2'-O-(t-butyldimethylsilyl)aden osine (1.77 g, 23.0 µmol) was placed in a stainless-steel column, and a nucleic acid autosynthesizer (AKTAoligopilot plus 10: manufactured by GE Healthcare Bioscience) was used to perform the synthesis according to the sequence of the designated nucleic acid, and thereafter the protecting group at the 5'-end was removed and the entire stainless-steel column was dried for 5 hours using a vacuum pump to obtain 1.79 g of CPG solid support.

As nucleic acid monomer compounds, N⁶-acetyl-5'-O-(4,4'-dimethoxytrityl)-2'-O-(2-cyanoethoxymethyl)adenos ine 3'-O-(2-cyanoethyl N,N-diisopropylphosphoramidite) and 5'-O-(4,4'-dimethoxytrityl)-2'-O-(2-cyanoethoxymethyl)uridine 3' -O-(2-cyanoethyl N,N-diisopropylphosphoramidite) produced according to the method described in International Publication 2008/050670 were employed. 5-Benzylmercapto-1H-tetrazole as a condensation catalyst, an iodine solution as an oxidizer, and phenoxyacetic anhydride and N-methylimidazole/2,6-lutidine solution as capping solutions were employed.

The CPG solid support obtained (621 mg, 8.0 µmol) was placed in a polypropylene column equipped with a filter (Libra Tube: manufactured by HiPep Laboratories), washed with dehydrated acetonitrile (3 x 5 mL) under an argon atmosphere, dried for 1 hour using a vacuum pump, and to the residue was added 5-benzylmercapto-1H-tetrazole (246 mg, 1.28 mmol) and a 0.1 M solution of bis(2-cyanoethoxy)-N,N-diisopropylaminophosphine in acetonitrile (3.2 mL,320 µmol), and reacted for 10 minutes at room temperature. After removal of the reaction solution, 5-benzylmercapto-1H-tetrazole (246 mg, 1.28 mmol) and a 0.1 M solution of bis(2-cyanoethoxy)-N,N-diisopropylaminophosphine in acetonitrile (3.2 mL,320 µmol) were added again and reaction was performed for 10 minutes. Dehydrated pyridine (3 x 5mL) was used to wash the solid support, a 0.05 M solution of iodine in pyridine (containing 10% H₂O, 3 mL) was used for oxidization, and washing was performed with dehydrated pyridine (3 x 5 mL) and dehydrated dichloromethane, (3 x 5 mL) in that order, and then a 0.2 M solution of phenoxyacetic anhydride in acetonitrile (0.5 mL) and a 20% solution of N-methylimidazole/30% 2,6-lutidine in acetonitrile (0.5 mL) were used for capping. Dehydrated dichloromethane (3 × 5 mL) was used for washing, an N,O-bis(trimethylsilyl)acetamide-pyridine solution mixture (1:1 (v/v), 4.0 mL) were used for performing a pretreatment for 20 minutes, and then, 1,8-diazabicyclo[5.4.0]-7-undecene (800 µL) were added and the mixture was reacted for 10 minutes. The solid support obtained was washed with dehydrated pyridine (3 x 5 mL) and dehydrated dichloromethane (3 x 5 mL) in that order, and dried using a vacuum pump for 15 minutes. A triethylamine-methanol solution mixture (1:9 (v/v), 5.0 mL) was added and the mixture was reacted for 3 hours at room temperature. The solid support obtained was washed with dehydrated acetonitrile (3 x 5 mL) and then dried using a vacuum pump for 3 hours to obtain 614 mg of CPG solid support.

In order to identify the sample, a small amount of the CPG solid support (15.3 mg, 0.2 µmol) was used to perform the aftertreatment. As a cleaving agent, a concentrated aqueous ammonia-ethanol mixture was used to perform the cleavage from the CPG solid support over a period of 1 hour at 40°C and the removal of the protecting groups of the nucleic acid bases. The reaction mixture was filtered and the filtrate was concentrated under reduced pressure. To the residue was added a 0.67 M solution of tetrabutylammonium fluoride in DMSO (0.75 mL) containing 0.67% nitromethane and reacted for 3 hours at room temperature to remove the protecting group of the 2'-hydroxyl group. To the reaction solution was added 1 M Tris HCl buffer solution (0.5 mL) under cooling with an ice bath, and this solution was added dropwise to ethanol (12 mL), and the precipitate obtained was subjected to HPLC (ion-exchange column) to measure the purity. The purity of the derivative monophosphorylated at the 5'-end was 90.9%. The molecular weight of the obtained derivative monophosphorylated at the 5'-end was measured by MALDI-TOF-MS.
MALDI-TOF-MS: Calculated: 982.60
Found: 983.38

### Example 1

### 6-Trifluoromethylbenzotriazol-1-yl bis(2-cyanoethyl)phosphate

1-Hydroxy-6-trifluoromethylbenzotriazol (32.5 mg, 160 µmol) was dried using a vacuum pump for 1 hour. Under an argon atmosphere, a 0.1 M solution of bis(2-cyanoethoxy)-N,N-diisopropylaminophosphine in acetonitrile (320 µL, 32 µmol) was added, and the reaction mixture was stirred for 1 hour at room temperature to produce the desired compound. ³¹P-NMR (CDCl₃) δ (ppm): -1.23

### Example 2

### Production of 3-mer (AUA) pyrophosphorylated at the 5'-end (SEQ ID NO: 1)

The pre-cleavage CPG solid support (30.7 mg, 0.4 µmol) of the 3-mer (AUA) monophosphorylated at the 5'-end (SEQ ID NO: 1) produced in Reference Example 1 was placed in a polypropylene column equipped with a filter (Libra Tube: manufactured by HiPep Laboratories) and subjected to an argon atmosphere. An acetonitrile solution of the compound produced in Example 1 was added to the solid support in the Libra Tube, and to the mixture was added 1-methyl-2-pyrrolidone (36 µL) and the mixture was reacted for 10 minutes at room temperature. Dehydrated pyridine (3 x 1 mL) and dehydrated dichloromethane (3 x 1 mL) were used for washing in that order, and N,O-bis(trimethylsilyl)acetamide-pyridine solution mixture (1:1 (v/v), 360 µL) was used for pretreatment for 20 minutes, and then 1,8-diazabicyclo[5.4.0]-7-undecene (72 µL) was added, and the mixture was reacted for 10 minutes. The solid support obtained was washed with dehydrated pyridine (3 × 1 mL) and dehydrated dichloromethane (3 x 1 mL) in that order, and then dried using a vacuum pump for 15 minutes. 30.2 mg of the CPG solid support was obtained.

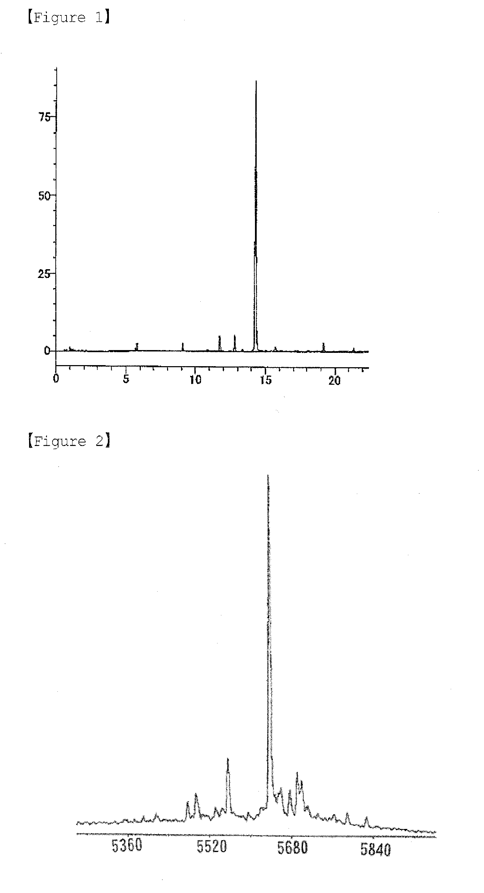

As a cleaving agent, a concentrated aqueous ammonia-ethanol mixture was used to perform the cleavage from the CPG solid support over a period of 1 hour at 40°C and the removal of the protecting groups of the nucleic acid bases. The reaction mixture was filtered and the filtrate was concentrated under reduced pressure. To the residue was added a 0.67 M solution of tetrabutylammonium fluoride in DMSO (0.75 mL) containing 0.67% nitromethane and the mixture was reacted for 2 hours at room temperature to remove the protecting group of the 2'-hydroxyl group. To the reaction solution was added 1 M Tris HCl buffer solution (0.5 mL) under cooling with an ice bath, and this solution was added dropwise to ethanol (12 mL), and the precipitate obtained was subjected to HPLC (ion-exchange column) to measure the purity. The purity of the derivative pyrophosphorylated at the 5'-end was 76.0%. The purity of the derivative pyrophosphorylated at the 5'-end was divided by the purity of the derivative monophosphorylated at the 5'-end, and then multiplied by 100 to obtain the yield, which was 84%. The molecular weight of the obtained derivative pyrophosphorylated at the 5'-end was measured by MALDI-TOF-MS. The chromatogram obtained by HPLC analysis is shown in Figure 1.
MALDI-TOF-MS: Calculated: 1062.57
Found: 1063.46

### Example 3

### Benzotriazol-1-yl bis(2-cyanoethyl)phosphate

1-Hydroxybenzotriazole (21.6 mg, 160 µmol) was dried for 2 hours using a vacuum pump. Under an argon atmosphere, a 0.1 M solution of bis (2-cyanoethoxy)-N,N-diisopropylaminophosphine in acetonitrile (320 µL, 32 µmol) was added, and the reaction mixture was stirred for 1 hour at room temperature to produce the desired compound.

### Example 4

### Pyrophosphorylation of 3-mer (AUA) at the 5'-end (SEQ ID NO: 1)

The pre-cleavage CPG solid support (30.7 mg, 0.4 µmol) of the 3-mer (AUA) (SEQ ID NO: 1) monophosphorylated at the 5'-end produced in Reference Example 1 was placed in a polypropylene column equipped with a filter (Libra Tube: manufactured by HiPep Laboratories) and subjected to an argon atmosphere. A solution of the compound produced in Example 3 in acetonitrile was added to the solid support in the Libra Tube, and to the mixture was added 1-methyl-2-pyrrolidone (36 µL) and the mixture was reacted for 10 minutes at room temperature. The aftertreatment was performed by a method similar to that described in Example 2. The purity of the derivative pyrophosphorylated at the 5'-end was 55.5%. The purity of the derivative pyrophosphorylated at the 5'-end was divided by the purity of the derivative monophosphorylated at the 5'-send, and then multiplied by 100 to obtain the yield, which was 61%.

### Example 5

### 7-Aza-benzotriazol-1-yl bis(2-cyanoethyl)phosphate

1-Hydroxy-7-azabenzotriazole (21.8 mg, 160 µmol) was dried for 2 hours using a vacuum pump. Under an argon atmosphere, a 0.1 M solution of bis(2-cyanoethoxy)-N,N-diisopropylaminophosphine in acetonitrile (320 µL, 32 µmol) was added, and the reaction mixture was stirred for 1 hour at room temperature to produce the desired compound.

### Example 6

### Pyrophosphorylation of 3-mer (AUA) at the 5'-end

The pre-cleavage CPG solid support (30.7 mg, 0.4 µmol) of the 3-mer (AUA) (SEQ ID NO: 1) monophosphorylated at the 5'-end produced in Reference Example 1 was placed in a polypropylene column equipped with a filter (Libra Tube: manufactured by HiPep Laboratories) and subjected to an argon atmosphere. A solution of the compound produced in Example 5 in acetonitrile was added to the solid support in the Libra Tube, and to the mixture was added 1-methyl-2-pyrrolidone (36 (µL) and the mixture was reacted for 10 minutes at room temperature. The aftertreatment was performed by a method similar to that described in Example 2. The purity of the derivative pyrophosphorylated at the 5'-end was 71.3%. The purity of the derivative pyrophosphorylated at the 5'-end was divided by the purity of the derivative monophosphorylated at the 5'-end, and then multiplied by 100 to obtain the yield, which was 78%.

### Example 7

### 6-Trifluoromethylbenzotriazol-1-yl

### (2-cyanoethyl)-2-[2'-O-(4,4'-dimethoxytrityloxy)ethylsulfonyl]ethyl phosphate

1-Hydroxy-6-trifluoromethylbenzotriazol (32.5 mg, 160 µmol) was dried using a vacuum pump for 1 hour. Under an argon atmosphere, a 0.1 M solution of (2-cyanoethoxy)-2-[2'-O-(4,4'-dimethoxytrityloxy)ethylsulfonyl]ethoxy-N,N-diisopropylaminophosphine in acetonitrile (320 µL, 32 µmol) was added, and the reaction mixture was stirred for 2 hours at room temperature.

### Example 8

### Pyrophosphorylation of 3-mer (AUA) at the 5'-end (SEQ ID NO: 1)

The pre-cleavage CPG solid support (30.7 mg, 0.4 µmol) of the 3-mer (AUA) (SEQ ID NO: 1) monophosphorylated at the 5'-end produced in Reference Example 1 was placed in a polypropylene column equipped with a filter (Libra Tube: manufactured by HiPep Laboratories) and subjected to an argon atmosphere. A solution of the compound produced in Example 7 in acetonitrile was added to the solid support in the Libra Tube, 1-methyl-2-pyrrolidone (36 µL) was added, and the mixture was reacted for 30 minutes at room temperature. The aftertreatment was performed by a method similar to that described in Example 2. The purity of the derivative pyrophosphorylated at the 5'-end was 65.0%. The purity of the derivative pyrophosphorylated at the 5'-end was divided by the purity of the derivative monophosphorylated at the 5'-end, and then multiplied by 100 to obtain the yield, which was 72%.

### Example 9

### Pyrophosphorylation of 122-mer nucleic acid at the 5'-end 122-Mer nucleic acid:

5′-GCCGCCGCCACCAUGGGACAUGCUGAAGGGACAUUUACCAGUGAUGUAAGUUCUUAUUUGGAAGGCC

AAGCUGCCAAGGAAUUCAUUGCUUGGCUGGUGAAAGGCCGAGGAUAACUAUAUCA-3′ (SEQ ID NO: 2)

### Step 1

A commercially available CPG solid support derivatized with N⁶-benzoyl-5'-O-(4,4'-dimethoxytrityl)-2'-O-(t-butyldimethylsilyl) aden osine (330.7 g, 4.3 µmol) was placed in a stainless-steel column, and a nucleic acid autosynthesizer (AKTAoligopilot plus 10: manufactured by GE Healthcare Bioscience) was employed to perform the synthesis according to the sequence of the designated nucleic acid, and thereafter the phosphorylating reagent was allowed to effect condensation once. The 5'-terminal protecting group was removed and the entire stainless-steel column was dried for 5 hours using a vacuum pump. 562 mg of the CPG solid support was obtained.

As nucleic acid monomer compounds, N⁶-acetyl-5'-O-(4,4'-dimethoxytrityl)-2'-O-(2-cyanoethoxymethyl) adenos ine 3'-O-(2-cyanoethyl N,N-diisopropylphosphoramidite), N²-phenoxyacetyl-5'-O-(4,4'-dimethoxytrityl)-2'-O-(2-cyanoethoxymethyl )guanosine 3'-O-(2-cyanoethyl N,N-diisopropylphosphoramidite), N⁴-acetyl-5'-O-(4,4'-dimethoxytrityl)-2'-O-(2-cyanoethoxymethyl) cytidi ne 3'-O-(2-cyanoethyl N,N-diisopropylphosphoramidite), 5'-O-(4,4'-dimethoxytrityl)-2'-O-(2-cyanoethoxymethyl) uridine 3'-O- (2-cyanoethyl N,N-diisopropylphosphoramidite) produced according to the method described in International Publication 2008/050670 were employed. As a phosphorylating reagent, (2-cyanoethoxy)-2-(2'-O-4,4'-dimethoxytrityloxyethylsulfonyl)ethoxy-N, N-diisopropylaminophosphine was employed. 5-Benzylmercapto-1H-tetrazole as a condensation catalyst, an iodine solution as an oxidizer, and phenoxyacetic anhydride and N-methylimidazole/2,6-lutidine solution as capping solution were employed.

The CPG solid support obtained (403 mg, 3.1 µmol) was placed in a polypropylene column equipped with a filter (Libra Tube: manufactured by HiPep Laboratories), dehydrated dichloromethane (2 x 3 mL) was used for washing under an argon atmosphere, N,O-bis(trimethylsilyl(acetamide)-pyridine solution mixture (1:1 (v/v), 3.6 mL) was used for pretreatment for 20 minutes, and then 1,8-diazabicyclo [5.4.0] -7-undecene (720 µL) was added, and the mixture was reacted for 10 minutes. The resultant solid support was washed with dehydrated pyridine (3 × 3 mL) and dehydrated dichloromethane (3 × 3 mL) in that order, and then dried using a vacuum pump for 15 minutes. To the solid support was added a triethylamine-methanol solution mixture (1:9 (v/v), 4.0 mL) and it was reacted for 3 hours at room temperature. The CPG solid support obtained was washed with dehydrated acetonitrile (4 mL × 3), and then dried for 3 hours using a vacuum pump. 397 mg of the CPG solid support was obtained.

In order to identify the sample, a small amount of the CPG solid support (25.6 mg, 0.2 µmol) was used to perform the aftertreatment. As a cleaving agent, a concentrated aqueous ammonia-ethanol mixture was used to perform a cleavage from the CPG solid support and the removal of the protecting groups of the nucleic acid bases over a period of 15 hours at 35°C. The reaction mixture was filtered and the filtrate was concentrated under reduced pressure. To the residue was added a 0.67 M solution of tetrabutylammonium fluoride in DMSO (1.5 mL) containing 0.67% nitromethane and the mixture was reacted for 5 hours and 30 minutes at room temperature to remove the protecting group of the 2'-hydroxyl group. To the reaction solution was added 1 M Tris HCl buffer solution (1.0 mL) under cooling with an ice bath, and this solution was added dropwise to ethanol (40 mL). The precipitate obtained was dissolved in water (2 mL), and MazF, (manufactured by TAKARA BIO INC.), an enzyme that selectively cleaves at ACA sequences, was used to cleave the 17-mer nucleic acid from the 5'-terminus (5'-GCCGCCGCCACCAUGGG-3' (SEQ ID NO: 3). The molecular weight of the obtained 17-mer nucleic acid monophosphorylated at the 5'-end was measured by MALDI-TOF-MS.
MALDI-TOF-MS: Calculated: 5557.33
Found: 5558.22

### Step 2

1-Hydroxybenzotriazole (21.6 mg, 160 µmol) was dried for 30 minutes using a vacuum pump. Under an argon atmosphere, 0.1 M solution of bis(2-cyanoethoxy)-N,N-diisopropylaminophosphine in acetonitrile (320 µL, 32 µmol) was added, and the reaction mixture was stirred for 1 hour at room temperature to produce benzotriazol-1-yl bis(2-cyanoethyl)phosphate.

### Step 3

The pre-cleavage CPG solid support (25.7 mg, 0.2 µmol) of the 122-mer nucleic acid monophosphorylated at the 5'-end was placed in a polypropylene column equipped with a filter (Libra Tube: manufactured by HiPep Laboratories) and subjected to an argon atmosphere. The solution of benzotriazol-1-yl bis(2-cyanoethyl)phosphate produced in Step 2 in acetonitrile was added to the solid support in the Libra Tube, and to the mixture was added 1-methyl-2-pyrrolidone (36 µL) and the mixture was reacted for 10 minutes at room temperature. Dehydrated pyridine (3 × 1 mL) and dehydrated dichloromethane (3 × 1 mL) were used for washing in that order, N,O-bis(trimethylsilyl)acetamide-pyridine solution mixture (1:1 (v/v), 360 µL) was used for pretreatment for 20 minutes, and then 1,8-diazabicyclo[5.4.0]-7-undecene (72 µL) was added, and the mixture was reacted for 10 minutes. The resultant solid support was washed with dehydrated pyridine (3 × 1 mL) and dehydrated dichloromethane (3 × 1 mL) in that order, and then dried using a vacuum pump for 15 minutes. 23.0 mg of the CPG solid support was obtained.

As a cleaving agent, a concentrated aqueous ammonia-ethanol mixture was used to perform a cleavage from the CPG solid support and the removal of the protecting groups of the nucleic acid bases over a period of 15 hours at 35°C. The reaction mixture was filtered and the filtrate was concentrated under reduced pressure. To the residue was added a 0.67 M solution of tetrabutylammonium fluoride in DMSO (1.5 mL) containing 0.67% nitromethane and the mixture was reacted for 5 hours at room temperature to remove the protecting group of the 2'-hydroxyl group. To the reaction solution was added 1 M Tris HCl buffer solution (1.0 mL) under cooling with an ice bath, and this solution was added dropwise to ethanol (40 mL) to obtain a precipitate. The precipitate obtained was dissolved in water (2 mL), and MazF (manufactured by TAKARA BIO INC.), an enzyme that selectively cleaves at ACA sequences, was used to cleave the 17-mer nucleic acid from the 5'-terminus. The molecular weight of the resultant derivative 17-mer nucleic acid pyrophosphorylated at the 5'-end was measured by MALDI-TOF-MS. The spectrum obtained by MALDI-TOF-MS is shown in Figure 2.
MALDI-TOF-MS: Calculated: 5637.30
Found: 5639.12

### Comparative 1

### Production of 3-mer (AUA) pyrophosphorylated at the 5'-end (SEQ ID NO: 1)

### Step 1

Under an argon atmosphere, to S,S-diphenylphosphorodithioate cyclohexylammonium salt (1.25 g, 3.29 mmol) was added dehydrated pyridine (15 mL) to dissolve, and the solvent was distilled off. This azeotropic procedure was repeated, and drying was performed for 30 minutes using a vacuum pump. Under an argon atmosphere, to the residue was added dehydrated pyridine (20 mL) to dissolve, and then was added mesitylenedisulfonyl dichloride (1.39 g, 4.38 mmol) and the mixture was stirred for 50 minutes at room temperature.

This reaction solution was added to 2-{2-(4,4'-dimethoxytrityl)ethylsulfonyl} ethanol (1.00 g, 2.19 mmol) in a recovery flask which was provided separately, and stirred for 2 hours at room temperature. After completion of the reaction, to the reaction solution was added water, and the mixture was partitioned with ethyl acetate, and then the organic layer was washed with water and then washed with saturated brine. The organic layer was dried over anhydrous sodium sulfate and filtered, and then the solvent was distilled off. The residue was purified by a silica gel column chromatography (eluent: hexane, ethyl acetate) to obtain 2-(2'-O-4,4'-dimethoxytrityloxyethylsulfonyl)ethyl S,S-diphenylphosphorodithioate (1.21 g, 77%).
¹H-NMR(CDCl₃)δ(ppm): 3.08(t,2H,J=5.6Hz); 3.49(t,2H,J=6.0Hz); 3.60(t,2H,J=5.6Hz); 3.78(s,6H); 4.65(dt,2H,J=6.0Hz,8.7Hz); 6.82-6.86(m,4H); 7.20-7.44(m,15H); 7.52-7.56(m,4H)
³¹P-NMR(CDCl₃)δ(ppm): 53.1

Under an argon atmosphere, to a 5 M hypophosphorous acid (10.9 g, 52.6 mmol) was added dehydrated pyridine (15 mL) to dissolve, and the solvent was distilled off. This azeotropic procedure was performed a further 3 times, and drying was performed for 40 minutes using a vacuum pump. Under an argon atmosphere, to the residue was added dehydrated pyridine (13 mL) to dissolve, and to the mixture was added triethylamine (6. 01 mL, 43.1 mmol, dried with molecular sieve 5A) and the mixture was stirred for several minutes at room temperature. This reaction solution was added to 2-(2'-O-4,4'-dimethoxytrityloxyethylsulfonyl)ethyl S,S-diphenylphosphorodithioate (1.15 g, 1.60 mmol, distilled azeotropically with dehydrated pyridine) in a recovery flask provided separately, and stirred for 1 hour at 35°C. After completion of the reaction, to the reaction solution was added pyridine-iced water (1:1 (v/v), 150 mL), and further added water (100 mL), and the mixture was partitioned with hexane. The aqueous layer was washed twice with hexane, and the aqueous layer was extracted 3 times with dichloromethane. The organic layer was dried over anhydrous sodium sulfate and filtered, and then the solvent was distilled off. The residue was purified twice by a silica gel column chromatography (eluent: dichloromethane, methanol). The residue was dissolved in dichloromethane, and partitioned twice with a 1 M aqueous solution of triethylammonium bicarbonate. The organic layer was dried over anhydrous sodium sulfate and filtered, and then the solvent was distilled off to obtain S-phenyl (2'-O-4,4'-dimethoxytrityloxyethylsulfonyl) ethyl 2-phosphorothioate triethylammonium salt (567 mg, 49%).
¹H-NMR(Pyridine-d5)δ(ppm): 1.10(t,9H,J=7.2Hz); 2.86(q,6H,J=7.2Hz); 3.66(s,6H); 3.70(t,2H,J=5.2Hz); 3.79(t,2H,J=5.2Hz); 3.88(t,2H,J=5.8Hz); 4.88(dt,2H,J=5.8Hz,8.5Hz); 5.42(bs,1H); 6.95-8.06(m,18H)
³¹P-NMR(Pyridine-d5)δ(ppm): 14.9

### Step 2

S-Phenyl (2'-O-4,4'-dimethoxytrityloxyethylsulfonyl)ethyl 2-phosphorothioate triethylammonium salt (23.4 mg, 32 µmol) was dissolved in dehydrated pyridine (2 mL), and the solvent was distilled off. This azeotropic procedure was performed again, and drying was performed for 30 minutes using a vacuum pump. Under an argon atmosphere, to the residue was added dehydrated pyridine (0.8 mL) to dissolve, and then added 1-hydroxy-4-nitro-6-trifluoromethylbenzotriazol (23.8 mg, 96 µmol), followed by iodine (24.4 mg, 96 µmol), and then the mixture was stirred for 10 minutes at room temperature.

### Step 3

The pre-cleavage CPG solid support (30.7 mg, 0.4 µmol) of the 3-mer nucleic acid (AUA) (SEQ ID NO: 1) monophosphorylated at the 5'-end in Reference Example 1 was placed in a polypropylene column equipped with a filter (Libra Tube: manufactured by HiPep Laboratories), and then washed with dehydrated acetonitrile (2 × 5 mL) under an argon atmosphere, and then dried for 10 minutes using a vacuum pump. The solution prepared in Step 2 was added to the solid support in the Libra Tube, and the mixture was reacted for 10 minutes at room temperature. The solid support was washed with dehydrated pyridine (3 × 1 mL) and dehydrated dichloromethane (3 x 1 mL) in that order, and the 4,4'-dimethoxytrityl group was removed by a 3% solution of dichloroacetic acid in toluene (3 × 1 mL). The solid support was washed with dehydrated dichloromethane (3 × 1 mL) , and pretreated with an N,O-bis(trimethylsilyl)acetamide-pyridine solution mixture (1:1 (v/v), 360 µL) for 20 minutes, and then to it was added 1,8-diazabicyclo[5.4.0]-7-undecene (72 µL), and reacted for 10 minutes. The solid support obtained was washed with dehydrated pyridine (3 × 1 mL) and dehydrated dichloromethane (3 × 1 mL) in that order, and then dried using a vacuum pump for 15 minutes. 30.2 mg of the CPG solid support was obtained.

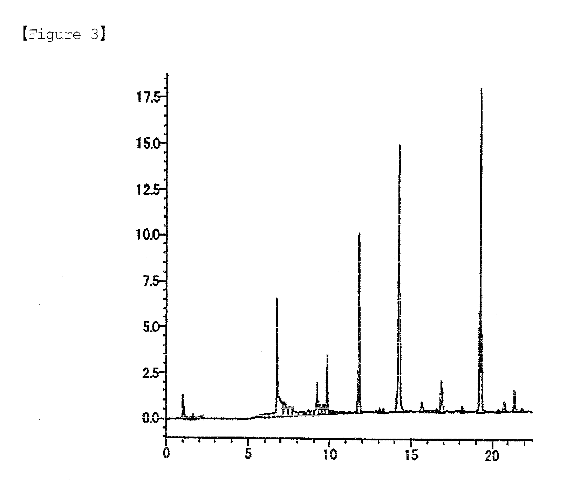

As a cleaving agent, a concentrated aqueous ammonia-ethanol mixture was used to perform the cleavage from the CPG solid support and the removal of the protecting groups of the nucleic acid bases over a period of 1 hour at 40°C. The reaction mixture was filtered and the filtrate was concentrated under reduced pressure. To the residue was added a 0.67 M solution of tetrabutylammonium fluoride in DMSO (0.75 mL) containing 0.67% nitromethane, and the mixture was reacted for 2.5 hours at room temperature to remove the protecting group of the 2'-hydroxyl group. To the reaction solution was added 1 M Tris HCl buffer solution (0.5 mL) under cooling with an ice bath, and this solution was added dropwise to ethanol (12 mL), and the purity of the resultant precipitate was measured by HPLC (ion-exchange column). The purity of the derivative pyrophosphorylated at the 5'-end was 20.7%. The purity of the derivative pyrophosphorylated at the 5'-end was divided by the purity of the derivative monophosphorylated at the 5'-end, and then multiplied by 100 to obtain the yield, which was 23%. The chromatogram obtained by the HPLC analysis is shown in Figure 3.

## Claims

1. A compound represented by the following formula (A): 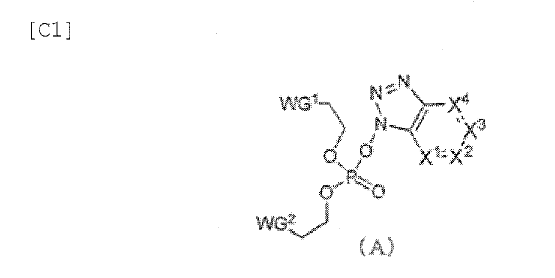 wherein WG¹ and WG² are the same or different and each represents cyano, nitro, halogen, alkylsulfonyl optionally substituted with hydroxy protected by a group represented by the following formula (1) or arylsulfonyl optionally substituted with 1 to 3 groups selected from the group consisting of halogen, alkyl, alkoxy, cyano and nitro; X¹, X², X³ and X⁴ are the same or different and each represents CR or N; and R represents H, methyl, halogen, trifluoromethyl, cyano, nitro, alkoxycarbonyl, carbamoyl, monoalkylcarbamoyl, dialkylcarbamoyl, sulfamoyl, monoalkylsulfamoyl, dialkylsulfamoyl or alkylsulfonyl; 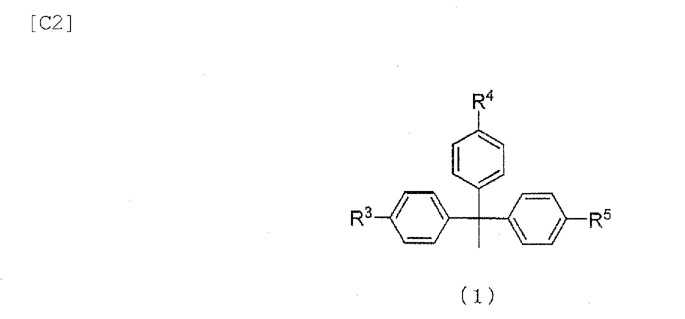 wherein R³, and R⁵ are the same or different and each represents hydrogen or alkoxy.

2. The compound according to Claim 1, wherein WG¹ and WG² are the same or different and each represents cyano or 2-(4,4'-dimethoxytrityloxy)ethylsulfonyl.

3. The compound according to any of Claims 1 and 2, wherein each of X¹, X², X³ and X⁴ is CH.

4. The compound according to any of Claims 1 and 2, wherein each of X¹, X³ and X⁴ is CH and X² is CR, and R is trifluoromethyl.

5. The compound according to any of Claims 1 and 2, wherein X¹ is N and each of X², X³ and X⁴ is CH.

6. A phosphorylating reagent comprising the compound according to any of Claims 1 to 5.

7. A method for producing a compound represented by the following formula (A), comprising reacting a compound represented by the following formula (2) with a compound represented by the following formula (3): 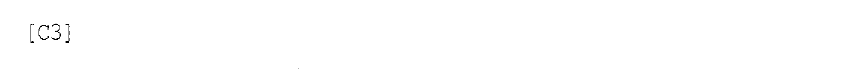 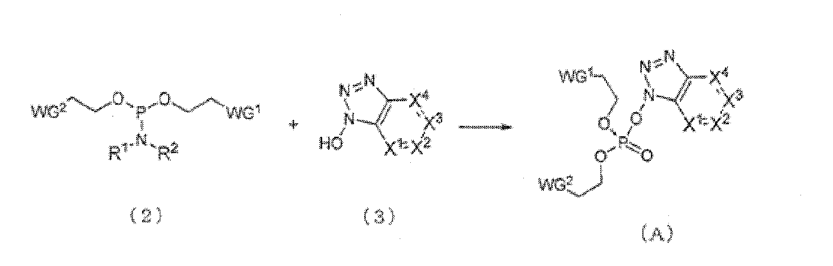 wherein WG¹ and WG² are the same or different and each represents cyano, nitro, halogen, alkylsulfonyl optionally substituted with hydroxy protected by a group represented by the following formula (1) or arylsulfonyl optionally substituted with 1 to 3 groups selected from the group consisting of halogen, alkyl, alkoxy, cyano and nitro; R¹ and R² are the same or different and each represents alkyl; X¹, X², X³ and X⁴ are the same or different and each represents CR or N; and R represents H, methyl, halogen, trifluoromethyl, cyano, nitro, alkoxycarbonyl, carbamoyl, monoalkylcarbamoyl, dialkylcarbamoyl, sulfamoyl, monoalkylsulfamoyl, dialkylsulfamoyl or alkylsulfonyl; 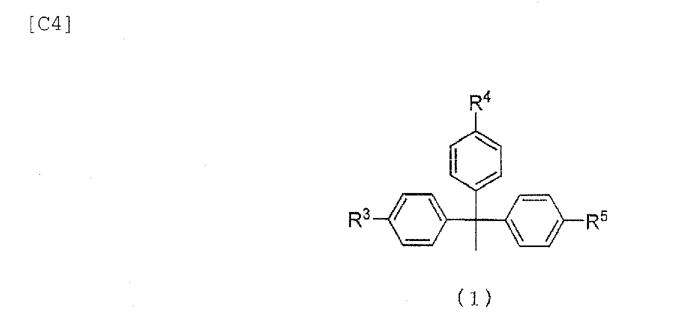 wherein R³, R⁴ and R⁵ are the same or different and each represents hydrogen or alkoxy.

8. The method according to Claim 7, wherein WG¹ and WG² are the same or different and each represents cyano or 2-(4,4'-dimethoxytrityloxy)ethylsulfonyl.

9. The method according to any of Claims 7 and 8, wherein each of X¹, X², X³ and X⁴ is CH.

10. The method according to any of Claims 7 and 8, wherein each of X¹, X³ and X⁴ is CH and X² is CR, and R is trifluoromethyl.

11. The method according to any of Claims 7 and 8, wherein X¹ is N and each of X², X³ and X⁴ is CH.

12. A method for producing a compound represented by the following formula (5), comprising reacting a compound represented by the following formula (4) with a compound represented by the following formula (A): 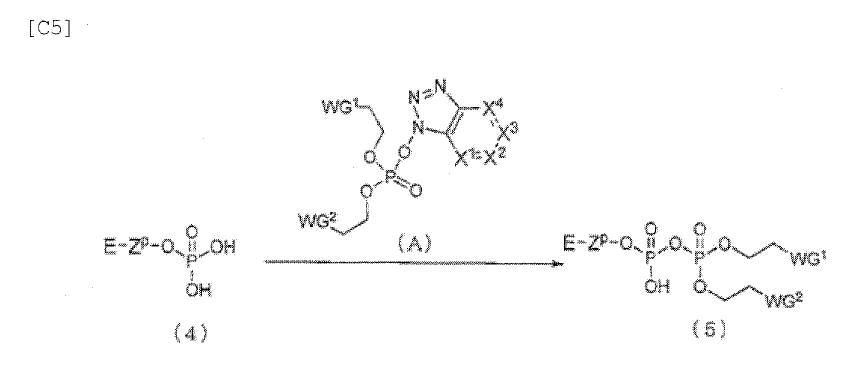 wherein E represents a solid support; Z^{p} represents an optionally protected nucleic acid; WG¹ and WG² are the same or different and each represents cyano, nitro, halogen, alkylsulfonyl optionally substituted with hydroxy protected by a group represented by the following formula (1) or arylsulfonyl optionally substituted with 1 to 3 groups selected from the group consisting of halogen, alkyl, alkoxy, cyano and nitro; X¹, X², X³ and X⁴ are the same or different and each represents CR or N; and R represents H, methyl, halogen, trifluoromethyl, cyano, nitro, alkoxycarbonyl, carbamoyl, monoalkylcarbamoyl, dialkylcarbamoyl, sulfamoyl, monoalkylsulfamoyl, dialkylsulfamoyl or alkylsulfonyl; 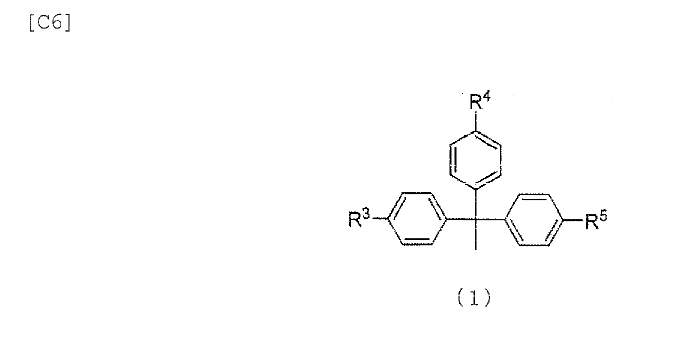 wherein R³, R⁴ and R⁵ are the same or different and each represents hydrogen or alkoxy.

13. The method according to Claim 12, wherein WG¹ and WG² are the same or different and each represents cyano or 2-(4,4'-dimethoxytrityloxy)ethylsulfonyl.

14. The method according to any of Claims 12 and 13, wherein each of X¹, X², X³ and X⁴ is CH.

15. The method according to any of Claims 12 and 13, wherein each of X¹, X³ and X⁴ is CH and X² is CR, and R is trifluoromethyl.

16. The method according to any of Claims 12 and 13, wherein X¹ is N and each of X², X³ and X⁴ is CH.

17. A method for producing a compound represented by the following formula (9), comprising the following Step 1 to Step 3: 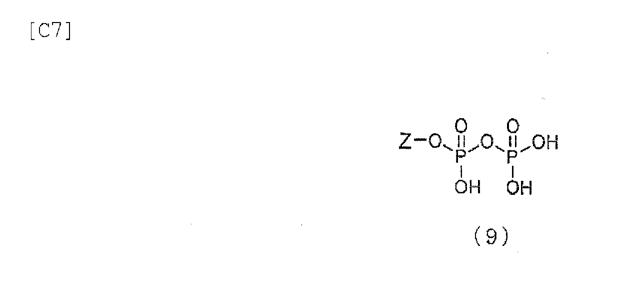 wherein Z represents a nucleic acid;
(Step 1) a step for reacting a compound represented by the following formula (4) with a compound represented by the following formula (A) to produce a compound represented by the following formula (5): 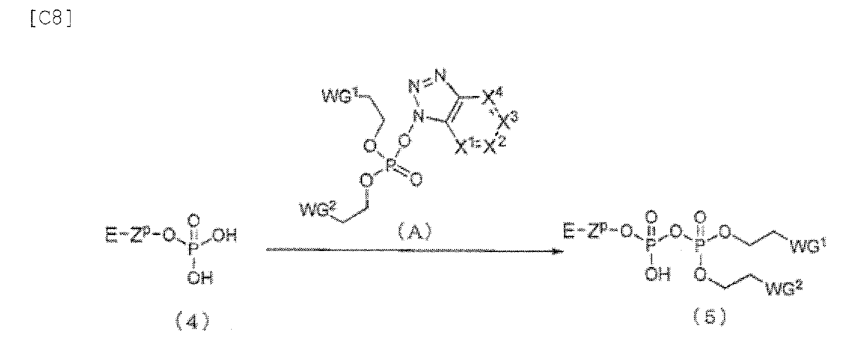 wherein E represents a solid support; Z^{p} represents an optionally protected nucleic acid; WG¹ and WG² are the same or different and each represents cyano, nitro, halogen, alkylsulfonyl optionally substituted with hydroxy protected by a group represented by the following formula (9) or arylsulfonyl optionally substituted with 1 to 3 groups selected from the group consisting of halogen, alkyl, alkoxy, cyano and nitro; X¹, X², X³ and X⁴ are the same or different and each represents CR or N; and R represents H, methyl, halogen, trifluoromethyl, cyano, nitro, alkoxycarbonyl, carbamoyl, monoalkylcarbamoyl, dialkylcarbamoyl, sulfamoyl, monoalkylsulfamoyl, dialkylsulfamoyl or alkylsulfonyl; 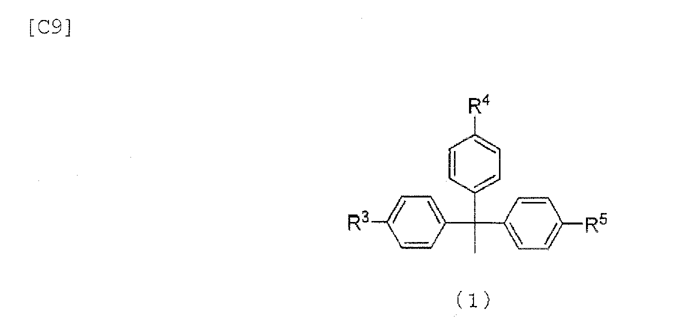 wherein R³, R⁴ and R⁵ are the same or different and each represents hydrogen or alkoxy;
(Step 2) a step for reacting Compound (5) with a silylating agent represented by the following formula (6) and a base to produce a compound represented by the following formula (7): 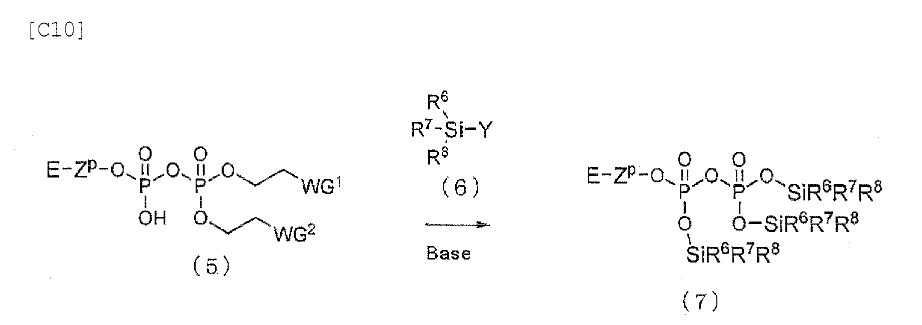 wherein WG¹, WG², E and Z^{p} are defined as described above; R⁶, R⁷ and R⁸ are the same or different and each represents alkyl, phenyl or naphthyl; and Y represents halogen, trifluoromethanesulfonyloxy or a group represented by the following formula (8): 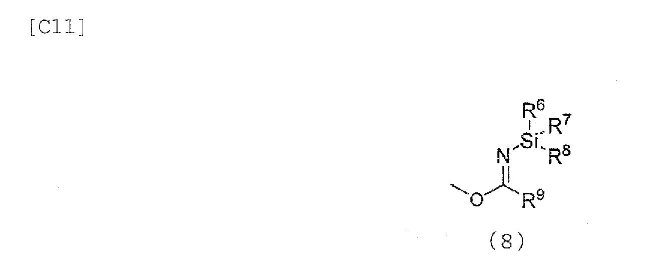 wherein R⁶, R⁷ and R⁸ are defined as described above; and R⁹ represents alkyl; (Step 3) a step for removing all protecting groups from Compound (7) to produce Compound (9); 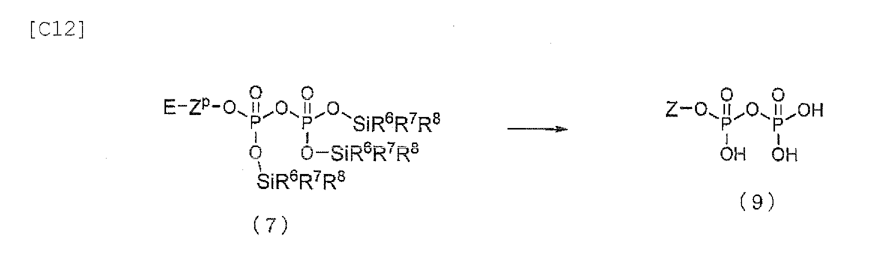 wherein E, R⁶, R⁷, R⁸ and Z^{p} are defined as described above; and Z represents a nucleic acid.

18. The method according to Claim 17, wherein WG¹ and WG² are the same or different and each represents cyano or 2-(4,4'-dimethoxytrityloxy)ethylsulfonyl.

19. The method according to any of Claims 17 and 18, wherein each of X¹, X², X³ and X⁴ is CH.

20. The method according to any of Claims 17 and 18, wherein each of X¹, X³ and X⁴ is CH and X² is CR, and R is trifluoromethyl. [Claim 21]
The method according to any of Claims 17 and 18, wherein X¹ is N and each of X², X³ and X⁴ is CH.


SEQUENCE LISTING

<110> The University of Tokyo

<110> Nippon Shinyaku Co., LTD.

<120> phosphorylating agent

<130> B-377N

<160> 3

<210> 1
<211> 3
<212> RNA
<213> Artificial sequence

<220>
<223> synthesized RNA

<400> 1
aua                                                                    3

<210> 2
<211> 122

<212> RNA

<213> Artificial sequence

<220>

<223> synthesized RNA

<400> 2

gccgccgcca ccaugggaca ugcugaaggg acauuuacca gugauguaag uucuuauuug 60

gaaggccaag cugccaagga auucauugcu uggcugguga aaggccgagg auaacuauau 120

ca 122

<210> 3

<211> 17

<212> RNA

<213> Artificial sequence

<220>

<223> synthesized RNA

<400> 3

Gccgccgcca ccauggg 17

SEQUENCE LISTING

<110> The University of Tokyo

<110> Nippon Shinyaku Co., LTD.

<120> phosphorylating agent

<130> B-377N

<160> 3

<210> 1
<211> 3
<212> RNA
<213> Artificial sequence

<220>
<223> synthesized RNA

<400> 1
aua 3

<210> 2
<211> 122
<212> RNA
<213> Artificial sequence

<220>
<223> synthesized RNA

<400> 2
gccgccgcca ccaugggaca ugcugaaggg acauuuacca gugauguaag uucuuauuug 60
gaaggccaag cugccaagga auucauugcu uggcugguga aaggccgagg auaacuauau 120
ca 122

<210> 3
<211> 17
<212> RNA
<213> Artificial sequence

<220>
<223> synthesized RNA

<400> 3
gccgccgcca ccauggg 17